# EUROPÄISCHE PATENTSCHRIFT

(11) **EP 0 401 840 B1**
(45) Veröffentlichungstag und Bekanntmachung des Hinweises auf die Patenterteilung: **07.05.1997**
(21) Anmeldenummer: 90110837.3
(22) Anmeldetag: 07.06.1990
(51) Int. Cl.: A61K 7/48, A61K 7/40, A61K 9/06

(54) **Hautschutzsalbe**
Skin protective ointment
Onguent pour la protection de la peau

(30) Priorität: 07.06.1989 DE 3918615
(43) Veröffentlichungstag der Anmeldung: 12.12.1990
(73) Patentinhaber: Physioderm GmbH & Co. KG, 63450 Hanau (DE)
(72) Erfinder: Nebel-Röhrig, F., Dipl.-Chem., D-6704 Mutterstadt (DE)
(74) Vertreter: Müller-Boré & Partner Patentanwälte

(56) Entgegenhaltungen:
- EP-A- 0 037 011
- WO-A-85/03641
- DE-A- 3 444 151
- FR-A- 1 563 677
- FR-A- 2 268 516
- US-A- 4 894 224
- PATENT ABSTRACTS OF JAPAN vol. 13, no. 3 (C-557)(3351) 6. Januar 1989

## Beschreibung

Die Erfindung betrifft eine Hautschutzsalbe auf der Basis einer wäßrigen Emulsion.

Um Abnutzungsdermatosen und allergischen Erkrankungen der Haut, vorallem der Hände, beim Arbeiten mit für die Haut schädlichen Substanzen, wie organischen Lösungsmitteln, Schmierfetten und Ölen, in Wasser gelösten Schadstoffen, wie Säuren und Laugen, Tensiden, verschiedenen Schwermetallsalzen, wassergemischten Bohrölemulsionen oder dergleichen, vorzubeugen, ist es bekannt, prophylaktisch Hautschutzsalben auf die Haut aufzutragen.

Als Hautschutzsalben sind einerseits hydrophob aufgebaute Salben mit einer Schutzwirkung gegen wäßrige oder in Wasser gelöste Schadstoffe sowie andererseits hydrophil eingestellte Hautschutzsalben mit einer Schutzwirkung gegen nichtwäßrige oder wasserunlösliche Schadstoffe bekannt. Diese Hautschutzsalben haben jedoch den Nachteil, daß sie jeweils nur gegen eine der beiden genannten Schadstoffgruppen schützen, so daß, je nach den in Frage kommenden Schadstoffen, verschiedene Hautschutzsalben angewendet werden müssen, was dann unzweckmäßig ist, wenn die Art der Schadstoffe während bestimmter von einer Person durchzuführenden Arbeitsgänge wechselt.

Es sind deshalb Hautschutzsalben mit dem Ziel einer universellen Schutzwirkung gegenüber hydrophilen und hydrophoben Schadstoffen entwickelt worden, die jedoch den Nachteil besitzen, daß sie nicht gegenüber den beiden genannten Schadstoffen in gleichem Ausmaße wirksam sind. Beispielsweise sind Hautschutzsalben auf der Basis von Stearin bekannt, das mit einem wasserlöslichen Gelfilmbildner vermischt ist. Auf diese Weise wird die wasserabstoßene Wirkung von Stearin mit der Bildung eines in organischen Lösungsmitteln unlöslichen Gelfilms auf der Haut kombiniert. Damit diese Stearatsalben eine befriedigende Hautakzeptanz besitzen, müssen größere Mengen an Ölen und Fetten und/oder an Glykolen, Polyglykolen, sowie Glyzerin verwendet werden. Werden jedoch zu große Mengen an Ölen und Fetten verwendet, dann ist keine gute Schutzwirkung gegen Öle, Fette und organische Lösungsmittel gegeben, während andererseits ein zu hoher Zusatz an Glykolen, Polyglykolen und Glyzerin den Schutz gegenüber wäßrigen Schadstoffen verringert.

Bekannt sind ferner Hautschutzpräparate, die aus Spraydosen als Schaum auf die Haut aufgebracht werden. Sie besitzen zum Teil eine ziemlich gute Schutzwirkung gegenüber beiden Schadstoffgruppen, haben jedoch den Nachteil, daß sie nur mit Hilfe von Treibgasen aus entsprechenden Vorrichtungen, wie Spraydosen, aufgebracht werden können, was einerseits mit einem hohen Kostenaufwand verbunden ist und andererseits im Hinblick auf die Treibgase Probleme der Umweltbelastung und der Arbeitssicherheit aufwirft.

In der FR-A-2 268 516 wird eine Hautschutzsalbe beschrieben, die quellbare Cellulosederivate, Fettsäuren, flüssige Polysiloxane und Wasser als Komponenten enthält. Der Anteil der Fettsäuren und der Polysiloxane beträgt zusammen 0,35 bis 5 Gew.-%.

Die Erfindung hat sich daher die Aufgabe gestellt, unter Behebung der vorstehend geschilderten Nachteile eine Möglichkeit zu schaffen, die Haut mit doppelter Wirkung gegenüber hydrophilen und hydrophoben Schadstoffen zu schützen.

Diese Aufgabe wird durch eine Hautschutzsalbe auf der Basis einer wäßrigen Emulsion aus einer Kombination von
a) 0,1 bis 2 Gew.-% Cellulosederivaten, wobei die Cellulosederivate Methyl-, Hydroxyethyl-, Hydroxypropyl- oder Carboxymethylgruppen als Substituenten an einer oder mehreren der in der Glucoseeinheit der Cellulose enthaltenen freien Hydroxylgruppen enthalten,
b) 5 bis 30 Gew.-% einem oder mehreren hydrophoben Bestandteilen ausgewählt aus Stearinsäure, Stearaten, Palmitinsäure, Palmitaten, Myristinsäure, Myristaten, Walrat, Jobaöl, Kokosfettsäuremonoethanolamid und nichtsubstituierten langkettigen Paraffinölen,
c) 5 bis 35 Gew.-% zyklischen Polydialkylsiloxanen mit einem Molekulargewicht von 90 bis 1000, wobei die Alkylgruppen 1 bis 4 Kohlenstoffatome besitzen, oder geradkettigen oder verzweigten Alkanolen, Alkenolen oder Alkinolen,
d) 30 bis 75 Gew.-% Wasser,
wobei sich die Komponenten zu 100 Gew.-% ergänzen, gelöst.

In den Unteransprüchen sind vorteilhafte Ausführungsformen der Hautschutzsalbe beschrieben.

Die Erfindung beruht demgemäß auf der Erkenntnis, daß dann, wenn einer Zubereitung auf der Basis einer wäßrigen Emulsion aus überwiegend hydrophilen filmbildenden Bestandteilen a) und überwiegen hydrophoben Bestandteilen b) eine oder mehrere bei Körpertemperatur flüchtige flüssige Substanz(en) mit gegebenenfalls teilweise oberflächenaktivem Charakter zugesetzt wird, die erhaltene Zubereitung als Hautschutzsalbe mit einer guten Schutzwirkung gegenüber sowohl hydrophilen als auch hydrophoben Schadstoffen bei guter Hautakzeptanz verwendbar ist.

Durch die Verwendung dieser bei Körpertemperatur flüchtigen flüssigen Substanzen ist zur Erzielung einer guten Hautakzeptanz nicht mehr die Verwendung größerer Mengen von Ölen und Fetten und/oder Glykolen, Polyglykolen oder Glyzerin erforderlich.

Die erfindungsgemäß als Hautschutzsalbe eingesetzte Zubereitung zeigt ein äußerst angenehmes weiches Hautgefühl in Kombination mit einem sehr guten Hauteinziehvermögen, wobei weder der hydrophobe noch der hydrophile Charakter der Salbenbasis negativ verändert wird, so daß die erfindungsgemäßen Hautschutzsalben ausgezeichnete Schutzwirkungen sowohl gegenüber hydrophilen als auch gegenüber hydrophoben Schadstoffen beim Auftragen auf die Haut bewirken.

Die erfindungsgemäß als Hautschutzsalbe eingesetzte Zubereitung kann als Basis jede beliebige Mischung der dafür bekannten überwiegend hydrophilen filmbildenden Bestandteile a) und überwiegend hydrophoben Bestandteile b) in wäßriger Emulsion enthalten. Erfindungsgemäß als überwiegend hydrophile Bestandteile a) werden wasserlösliche und/oder wasserquellbare Substanzen, die einen Gelfilm zu bilden vermögen, eingesetzt, wobei wasserlösliche bzw. wasserquellbare filmbildende natürliche oder synthetische Hydrokolloide in Frage, kommen wie wasserlösliche bzw. wasserquellbare Zellulosederivate, d.h. solche mit Methyl-, Hydroxyethyl-, Hydroxypropyl- oder Carboxymethylgruppen als Substituenten an einer oder mehreren der in der Glukoseeinheit der Zellulose enthaltenen freien Hydroxylgruppen.

Als überwiegend hydrophobe Bestandteile b) kommen organische Verbindungen mit einer unverzweigten oder verzweigten Alkylkette mit mindestens 12 Kohlenstoffatomen, die 1 oder mehrere polare Gruppe(n) tragen können, in Frage. Diese Verbindungen enthalten 12 bis 24 Kohlenstoffatome und sind insbesondere aliphatischer Natur. Die gegebenenfalls vorliegenden polaren Gruppen können aus Hydroxylgruppen, Carbonsäuregruppen, Carbonsäureanionengruppen, Estergruppen, Ethergruppen, substituierten Amin- bzw. Amidgruppen oder dergleichen oder als Kombinationen davon bestehen, wobei die Substituenten der Amin- bzw. Amidgruppen aus kurzkettigen Alkylolresten mit insbesondere 1 bis 4 Kohlenstoffatomen bestehen können.

Als derartige Substanzen seien Stearinsäure, Stearate, wie Zinkstearat, Palmitinsäure, Palmitate, Myristinsäure, Myristate, Walrat, Jobaöl, Kokosfettsäuremonoethanolamid bzw. nichtsubstituierte langkettige Paraffinöle erwähnt.

Die bei Körpertemperatur (d.h. etwa 37°C) flüchtige flüssige Substanz c) mit gegebenenfalls teilweise oberflächenaktivem Charakter und gegebenenfalls einem Flammpunkt oberhalb Körpertemperatur muß natürlich ebenso wie die anderen Bestandteile der erfindungsgemäßen Hautschutzsalbe hautverträglich sein und besteht vorzugsweise aus linearen oder zyklischen Polydialkylsiloxanen mit niederem Molekulargewicht oder geradkettigen oder verzweigten Alkanolen, Alkenolen oder Alkinolen.

Die Polydialkylsiloxane besitzen vorzugsweise Molekulargewichte von 90 bis 1000, wobei die Alkylgruppen 1 bis 4 Kohlenstoffatome besitzen können. Es handelt sich um an sich bekannte Substanzen. Bevorzugt werden Dimethylpolysiloxane.

Die als Komponente c) eingesetzten Alkanole, Alkenole oder Alkinole besitzen vorzugsweise 2 bis 10 Kohlenstoffatome.

Die erfindungsgemäße Hautschutzsalbe enthält die Bestandteile a) in Mengen von 0,1 bis 2 Gew.-%, die Bestandteile b) in Mengen von 5 bis 30 Gew.-%, und die Bestandteile c) in Mengen von 5 bis 35 Gew.-%, jeweils als Aktivsubstanz gerechnet, sowie Wasser in Mengen von 30 bis 75 Gew.-%, wobei sich die angegebenen Mengen zu 100 Gew.-% ergänzen.

Außerdem kann die erfindungsgemäß als Hautschutzsalbe eingesetzte Zubereitung übliche in kosmetischen Cremes eingesetzte Zusätze enthalten, wie z.B. Pigmente, Farbstoffe, Duftstoffe, Konservierungsstoffe, den pH-Wert steuernde Mittel, wie Ammoniak oder Borax, Feuchtigkeitsregulatoren.

Die folgenden Beispiele erläutern die Erfindung:

### Beispiel 1

Die folgenden Komponenten wurden zu einer Hautschutzsalbe vermischt:

| | |
|---|---|
| Stearin (= Stearinsäure) | 12,00 % |
| Zinkstearat | 8,00 % |
| Paraffinöl | 2,50 % |
| Methylcellulose | 0,80 % |
| Cyklisches Dimethylpolysiloxan FP 54 ° | 20,00 % |
| Borax | 1,20 % |
| Ammoniak 25 %ig Konservierungsmittel Duftstoff | 0,57 % |
| Wasser dest. | Rest ad 100 % |

### Beispiel 2

Unter Einsatz der folgenden Bestandteile wurde eine Hautschutzsalbe durch Vermischen hergestellt:

| | |
|---|---|
| Stearin (Stearinsäure) | 20,00 % |
| Zinkstearat | 4,00 % |
| Paraffinöl | 3,50 % |
| Methylcellulose | 1,20 % |
| Cyklisches Dimethylpolysiloxan FP 54 ° | 30,00 % |
| Borax | 2,00 % |
| Ammoniak 25 %ig Konservierungsmittel Duftstoffe | 0,57 % |
| Wasser dest. | Rest ad 100 % |

Beide Salben zeigen eine gute Hautakzeptanz und waren prophylaktisch sehr wirksam sowohl gegenüber hydrophilen als auch hydrophoben Schadstoffen, wie durch dermatologische Tests bzw. Laborprüfungen ermittelt wurde.

## Patentansprüche (Patentansprüche für folgende(n) Vertragsstaat(en): AT, BE, CH, DE, FR, IT, LI, LU, NL)

1. Hautschutzsalbe auf der Basis einer wäßrigen Emulsion aus einer Kombination von
a) 0,1 bis 2 Gew.-% Cellulosederivaten, wobei die Cellulosederivate Methyl-, Hydroxyethyl-, Hydroxypropyl- oder Carboxymethylgruppen als Substituenten an einer oder mehreren der in der Glucoseeinheit der Cellulose enthaltenen freien Hydroxylgruppen enthalten,
b) 5 bis 30 Gew.-% einem oder mehreren hydrophoben Bestandteilen ausgewählt aus Stearinsäure, Stearaten, Palmitinsäure, Palmitaten, Myristinsäure, Myristaten, Walrat, Jobaöl, Kokosfettsäuremonoethanolamid und nichtsubstituierten langkettigen Paraffinölen,
c) 5 bis 35 Gew.-% zyklischen Polydialkylsiloxanen mit einem Molekulargewicht von 90 bis 1000, wobei die Alkylgruppen 1 bis 4 Kohlenstoffatome besitzen, oder geradkettigen oder verzweigten Alkanolen, Alkenolen oder Alkinolen,
d) 30 bis 75 Gew.-% Wasser,
wobei sich die Komponenten zu 100 Gew.-% ergänzen.

2. Hautschutzsalbe nach Anspruch 1, wobei zusätzlich in kosmetischen Salben übliche Additive enthalten sein können.

3. Hautschutzsalbe nach Anspruch 2, wobei die Additive ausgewählt sind aus Pigmenten, Farbstoffen, Duftstoffen, Konservierungsstoffen, pH-Wert steuernden Mitteln oder Feuchtigkeitsregulatoren.

4. Hautschutzsalbe nach einem der vorhergehenden Ansprüche, wobei das Cellulosederivat Methylcellulose ist.

5. Hautschutzsalbe nach einem der vorhergehenden Ansprüche, wobei das zyklische Polydialkylsiloxan zyklisches Dimethylpolysiloxan ist.

## Patentansprüche (Patentansprüche für folgende(n) Vertragsstaat(en): ES)

1. Verfahren zur Herstellung einer Hautschutzsalbe auf der Basis einer wäßrigen Emulsion, bei dem man die folgende Kombination von Komponenten miteinander vermischt:
a) 0,1 bis 2 Gew.-% Cellulosederivaten, wobei die Cellulosederivate Methyl-, Hydroxyethyl-, Hydroxypropyl- oder Carboxymethylgruppen als Substituenten an einer oder mehreren der in der Glucoseeinheit der Cellulose enthaltenen freien Hydroxylgruppen enthalten,
b) 5 bis 30 Gew.-% einem oder mehreren hydrophoben Bestandteilen ausgewählt aus Stearinsäure, Stearaten, Palmitinsäure, Palmitaten, Myristinsäure, Myristaten, Walrat, Jobaöl, Kokosfettsäuremonoethanolamid und nichtsubstituierten langkettigen Paraffinölen,
c) 5 bis 35 Gew.-% zyklischen Polydialkylsiloxanen mit einem Molekulargewicht von 90 bis 1000, wobei die Alkylgruppen 1 bis 4 Kohlenstoffatome besitzen, oder geradkettigen oder verzweigten Alkanolen, Alkenolen oder Alkinolen,
d) 30 bis 75 Gew.-% Wasser,
wobei sich die Komponenten zu 100 Gew.-% ergänzen.

2. Verfahren nach Anspruch 1, wobei zusätzlich in kosmetischen Salben übliche Additive enthalten sein können.

3. Verfahren nach Anspruch 2, wobei die Additive ausgewählt sind aus Pigmenten, Farbstoffen, Duftstoffen, Konservierungsstoffen, pH-Wert steuernden Mitteln oder Feuchtigkeitsregulatoren.

4. Verfahren nach einem der vorhergehenden Ansprüche, wobei das Cellulosederivat Methylcellulose ist.

5. Verfahren nach einem der vorhergehenden Ansprüche, wobei das zyklische Polydialkylsiloxan zyklisches Dimethylpolysiloxan ist.

## Claims (Claims for the following Contracting State(s): AT, BE, CH, DE, FR, IT, LI, LU, NL)

1. Skin protective ointment based on an aqueous emulsion consisting of a combination of
a) 0.1 to 2 % by weight of cellulose derivatives, where the cellulose derivatives contain methyl, hydroxyethyl, hydroxypropyl or carboxymethyl groups as substituents on one or more of the free hydroxyl groups present in the glucose unit of the cellulose,
b) 5 to 30 % by weight of one or more hydrophobic ingredients selected from stearic acid, stearates, palmitic acid, palmitates, myristic acid, myristates, spermaceti, jojoba oil, coconut fatty acid monoethanolamide and unsubstituted long-chain liquid paraffins,
c) 5 to 35 % by weight of cyclic polydialkylsiloxanes with a molecular weight of from 90 to 1000, where the alkyl groups have 1 to 4 carbon atoms, or straight-chain or branched alkanols, alkenols or alkynols,
d) 30 to 75 % by weight of water,
where the components total 100 % by weight.

2. Skin protective ointment according to Claim 1, where additives customary in cosmetic ointments may additionally be present.

3. Skin protective ointment according to Claim 2, where the additives are selected from pigments, dyes, perfumes, preservatives, pH-controlling agents or moisture regulators.

4. Skin protective ointment according to any of the preceding claims, where the cellulose derivative is methylcellulose.

5. Skin protective ointment according to any of the preceding claims, where the cyclic polydialkylsiloxane is cyclic dimethylpolysiloxane.

## Claims (Claims for the following Contracting State(s): ES)

1. Process for the production of a skin protective ointment based on an aqueous emulsion, comprising the following combination of components being mixed together:
a) 0.1 to 2 % by weight of cellulose derivatives, where the cellulose derivatives contain methyl, hydroxyethyl, hydroxypropyl or carboxymethyl groups as substituents on one or more of the free hydroxyl groups present in the glucose unit of the cellulose,
b) 5 to 30 % by weight of one or more hydrophobic ingredients selected from stearic acid, stearates, palmitic acid, palmitates, myristic acid, myristates, spermaceti, jojoba oil, coconut fatty acid monoethanolamide and unsubstituted long-chain liquid paraffins,
c) 5 to 35 % by weight of cyclic polydialkylsiloxanes with a molecular weight of from 90 to 1000, where the alkyl groups have 1 to 4 carbon atoms, or straight-chain or branched alkanols, alkenols or alkynols,
d) 30 to 75 % by weight of water,
where the components total 100 % by weight.

2. Process according to Claim 1, where additives customary in cosmetic ointments may additionally be present.

3. Process according to Claim 2, where the additives are selected from pigments, dyes, perfumes, preservatives, pH-controlling agents or moisture regulators.

4. Process according to any of the preceding claims, where the cellulose derivative is methylcellulose.

5. Process according to any of the preceding claims, where the cyclic polydialkylsiloxane is cyclic dimethylpolysiloxane.

## Revendications (Revendications pour l'(les) Etat(s) contractant(s) suivant(s): AT, BE, CH, DE, FR, IT, LI, LU, NL)

1. Onguent protecteur de la peau à base d'une émulsion aqueuse d'une combinaison de:
a) 0,1 à 2% en poids de dérivés de cellulose, dans lesquels lesdits dérivés de cellulose comportent des groupes méthyle, hydroxyéthyle, hydroxypropyle ou carboxyméthyle en tant que substituants sur un ou plusieurs des hydroxyles libres contenus dans les unités glucose de la cellulose,
b) 5 à 30% en poids d'un ou de plusieurs constituants hydrophobes choisis parmi acide stéarique, stéarates, acide palmitique, palmitates, acide myristique, myristates, blanc de baleine, huile de jojoba, monoéthanolamide d'acide gras de coco, et huile de paraffine à chaîne longue non substituée,
c) 5 à 35% en poids de polydialkylsiloxanes cycliques d'un poids moléculaire compris entre 90 et 1000, dans lesquels les groupes alkyle comportent 1 à 4 atomes de carbone ou d'alkanols, alkénols ou alkynols linéaires ou ramifiés,
d) 30 à 75% en poids d'eau,
la somme des constituants étant égale à 100% en poids.

2. Un onguent protecteur de la peau selon la revendication 1, dans lequel on peut incorporer les additifs habituels contenus dans les crèmes cosmétiques.

3. Un onguent protecteur de la peau selon la revendicatin 1, dans lequel les additifs sont choisis parmi les pigments, colorants, parfums, conservateurs, agents régulateurs du pH ou agents régulateurs de l'humidité.

4. Un onguent protecteur de la peau selon l'une quelconque des revendications précédentes, dans lequel le dérivé de cellulose est la méthylcellulose.

5. Un onguent protecteur de la peau selon l'une quelconque des revendications précédentes, dans lequel le polydialkylsiloxane cyclique est du diméthylpolysiloxane cyclique.

## Revendications (Revendications pour l'(les) Etat(s) contractant(s) suivant(s): ES)

1. Procédé de préparation d'un onguent protecteur de la peau à base d'une émulsion aqueuse d'une combinaison de:
a) 0,1 à 2% en poids de dérivés de cellulose, dans lesquels lesdits dérivés de cellulose comportent des groupes méthyle, hydroxyéthyle, hydroxypropyle ou carboxyméthyle en tant que substituants sur un ou plusieurs des hydroxyles libres contenus dans les unités glucose de la cellulose,
b) 5 à 30% en poids d'un ou de plusieurs constituants hydrophobes choisis parmi acide stéarique, stéarates, acide palmitique, palmitates, acide myristique, myristates, blanc de baleine, huile de jojoba, monoéthanolamide d'acide gras de coco, et huile de paraffine à chaîne longue non substituée,
c) 5 à 35% en poids de polydialkylsiloxanes cycliques d'un poids moléculaire compris entre 90 et 1000, dans lesquels les groupes alkyle comportent 1 à 4 atomes de carbone, ou d'alkanols, alkénols ou alkynols linéaires ou ramifiés,
d) 30 à 75% en poids d'eau,
la somme des constituants étant égale à 100% en poids.

2. Procédé selon la revendication 1, dans lequel on peut incorporer les additifs habituels contenus dans les crèmes cosmétiques.

3. Procédé selon la revendication 1, dans lequel les additifs sont choisis parmi les pigments, colorants, parfums, conservateurs, agents régulateurs du pH ou agents régulateurs de l'humidité.

4. Procédé selon l'une quelconque des revendications précédentes, dans lequel le dérivé de cellulose est la méthylcellulose.

5. Procédé selon l'une quelconque des revendications précédentes, dans lequel le polydialkylsiloxane cyclique est du diméthylpolysiloxane cyclique.
